(19) Europäisches Patentamt
European Patent Office
Office européen des brevets

(11) Veröffentlichungsnummer: **0 400 433 B1**

(12) **EUROPÄISCHE PATENTSCHRIFT**

(45) Veröffentlichungstag der Patentschrift: **10.11.93**

(51) Int. Cl.5: **C09B 11/12**, C07D 295/18, D21H 21/28

(21) Anmeldenummer: **90109548.9**

(22) Anmeldetag: **19.05.90**

# (54) Verdoppelte Triphenylmethanfarbstoffe sowie Piperazinderivate.

(30) Priorität: **31.05.89 DE 3917601**

(43) Veröffentlichungstag der Anmeldung:
**05.12.90 Patentblatt 90/49**

(45) Bekanntmachung des Hinweises auf die Patenterteilung:
**10.11.93 Patentblatt 93/45**

(84) Benannte Vertragsstaaten:
**CH DE DK FR GB IT LI SE**

(56) Entgegenhaltungen:
**EP-A- 0 022 520**
**FR-A- 2 401 958**

**JOURNAL OF MEDICAL CHEMISTRY, Band 11, Juli 1968, Seiten 801-804, American Chemical Society, Washington, US; T. IRIKURA et al.: "New analgetic agents. V. 1-Butyryl-4-cinnamylpiperazine hydrochloride and related compounds"**

(73) Patentinhaber: **BASF Aktiengesellschaft**
**Carl-Bosch-Strasse 38**
**D-67063 Ludwigshafen(DE)**

(72) Erfinder: **Hahn, Erwin, Dr.**
**Am Buechsenackerhang 31**
**D-6900 Heidelberg(DE)**
Erfinder: **Breitschaft, Walter, Dr.**
**Eichelbergstrasse 1**
**D-6800 Mannheim 1(DE)**
Erfinder: **Mayer, Udo, Dr.**
**Max-Slevogt-Strasse 27**
**D-6710 Frankenthal(DE)**
Erfinder: **Schroeder, Gunter-Rudolf, Dr.**
**Wredestrasse 71**
**D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Triphenylmethanfarbstoffe der Formel I

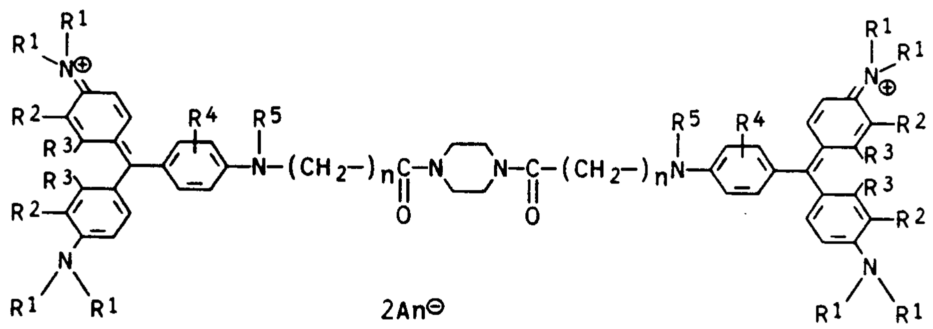

(I),

in der die Reste

$R^1$ gleich oder verschieden sind und unabhängig voneinander jeweils Wasserstoff oder gegebenenfalls substituiertes $C_1$-$C_6$-Alkyl oder zwei Reste $R^1$ zusammen mit dem sie verbindenen Stickstoffatom einen gesättigten 5- oder 6-gliedrigen heterocyclischen Rest, der gegebenenfalls weitere Heteroatome aufweisen kann,

$R^2$ und $R^5$ gleich oder verschieden sind und unabhängig voneinander jeweils Wasserstoff oder $C_1$-$C_4$-Alkyl,

$R^3$ und $R^4$ gleich oder verschieden sind und unabhängig voneinander jeweils Wasserstoff, $C_1$-$C_4$-Alkyl, $C_1$-$C_4$-Alkoxy oder Halogen,

$An^\ominus$ das Äquivalent eines Anions und

n gleich oder verschieden ist und 1 oder 2 bedeuten,

sowie Piperazinderivate als deren Zwischenprodukte.

Aus der DE-A-2 739 953 sind bereits verdoppelte Triphenylmethanfarbstoffe bekannt, die als Brückenglied z.B. den p-Xylylenrest oder den Piperazin-1,4-diylrest aufweisen. Bei der Herstellung derjenigen Farbstoffe, die über eine p-Xylylen-Brücke verfügen, muß man von 1,4-Bis(chlormethyl)benzol ausgehen, dessen Handhabbarkeit problematisch ist. Diejenigen Farbstoffe, die über eine Piperazin-1,4-diyl-Brücke verdoppelt sind, weisen noch Mängel in ihren anwendungstechnischen Eigenschaften auf.

Aufgabe der vorliegenden Erfindung war es daher, neue verdoppelte Triphenylmethanfarbstoffe bereitzustellen, die mittels einfacher Zwischenprodukte herzustellen sind und die über günstige anwendungstechnische Eigenschaften verfügen.

Demgemäß wurden die eingangs näher bezeichneten Triphenylmethanfarbstoffe der Formel I gefunden.

Wenn $R^1$ einen $C_1$-$C_6$-Alkylrest darstellt, der substituiert ist, können als Substituenten z.B. Hydroxy, $C_1$-$C_4$-Alkoxy, $C_1$-$C_4$-Alkanoyloxy, $C_1$-$C_4$-Alkoxycarbonyl, Cyano, Chlor, Acetyl, Acetylamino oder Phenyl in Betracht kommen.

Reste $R^1$, $R^2$, $R^3$, $R^4$ und $R^5$ sind z.B. Methyl, Ethyl, Propyl, Isopropyl, Butyl, Isobutyl, sec-Butyl oder tert-Butyl.

Reste $R^3$ und $R^4$ sind weiterhin z.B. Methoxy, Ethoxy, Propoxy, Isopropoxy, Butoxy, Isobutoxy, sec-Butoxy, Fluor, Chlor oder Brom.

Reste $R^1$ sind weiterhin z.B. Pentyl, Isopentyl, Neopentyl, tert-Pentyl, Hexyl, 2-Methylpentyl, 2-Hydroxyethyl, 2- oder 3-Hydroxypropyl, 2-Methoxyethyl, 2-Ethoxyethyl, 2- oder 3-Methoxypropyl, 2- oder 3-Ethoxypropyl, 2-Formyloxyethyl, 2-Acetyloyethyl, 2- oder 3-Formyloxypropyl, 2- oder 3-Acetyloxypropyl, 2-Methoxycarbonylethyl, 2-Ethoxycarbonylethyl, 2- oder 3-Methoxycarbonylpropyl, 2- oder 3-Ethoxycarbonylpropyl, 2-Cyanoethyl, 2- oder 3-Cyanopropyl, 2-Chlorethyl, 2- oder 3-Chlorpropyl, But-3-on-1-yl, Pent-4-on-1-yl, 2-Methylbut-3-on-1-yl, 2-Acetylaminoethyl, 2- oder 3-Acetylaminopropyl, Benzyl oder 1- oder 2-Phenylethyl.

Wenn zwei Reste $R^1$ zusammen mit dem sie verbindenen Stickstoffatom einen gesättigten 5- oder 6-gliedrigen heterocyclischen Rest bedeuten, der gegebenenfalls weitere Heteroatome aufweisen kann, so können als solche Reste z.B. Pyrrolidino, Piperidino, Morpholino, Thiomorpholino, Piperazino, N-($C_1$-$C_4$-Alkyl)piperazino, wie N-Methyl- oder N-Ethylpiperazino, in Betracht kommen.

Geeignete Anionen, von denen sich die Anionäquivalente $An^\ominus$ ableiten, sind z.B. Halogenid, wie Fluorid, Chorid, Bromid oder Iodid, Hydrogensulfat, Sulfat, Hydrogenphosphat, Phosphat, Borat, Tetrafluoroborat, Trichlorozinkat, Methosulfat, Ethosulfat, Benzolsulfonat, o- oder p-Toluolsulfonat, Methylsulfonat, Formiat,

Acetat, Propionat, Hydroxyacetat, Methoxyacetat oder Lactat.

Bevorzugt sind Triphenylmethanfarbstoffe der Formel I, in der die Reste

$R^1$ unabhängig voneinander Wasserstoff oder $C_1$-$C_4$-Alkyl oder 2-Hydroxyethyl,

$R^2$ unabhängig voneinander Wasserstoff oder Methyl,

$R^3$ und $R^4$ unabhängig voneinander Wasserstoff, Methyl, Methoxy, Ethoxy oder Halogen und

$R^5$ unabhängig voneinander Wasserstoff oder $C_1$-$C_4$-Alkyl bedeuten und $An^\ominus$ und n jeweils die obengenannte Bedeutung besitzen.

Besonders bevorzugt sind Triphenylmethanfarbstoffe der Formel I, in der n jeweils 1 bedeutet.

Insbesondere hervorzuheben sind Triphenylmethanfarbstoffe der Formel I, in der die Reste

$R^1$ unabhängig voneinander Wasserstoff, Methyl oder Ethyl,

$R^2$ unabhängig voneinander Wasserstoff oder Methyl,

$R^3$ unabhängig voneinander Wasserstoff oder Methoxy,

$R^4$ unabhängig voneinander Wasserstoff, Methyl, Methoxy oder Chlor,

$R^5$ unabhängig voneinander Wasserstoff, Methyl oder Ethyl und

n 1 bedeuten und

$An^\ominus$ die obengenannte Bedeutung besitzt.

Die Herstellung der erfindungsgemäßen Triphenylmethanfarbstoffe der Formel I kann nach an sich bekannten Methoden erfolgen. Beispielsweise kann man ein Piperazinderivat der Formel III

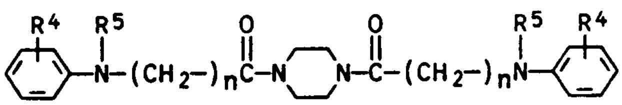 (III),

in der $R^4$, $R^5$ und n jeweils die obengenannte Bedeutung besitzen, mit einem Diphenylmethan der Formel IV

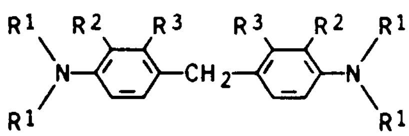 (IV),

in der $R^1$, $R^2$ und $R^3$ jeweils die obengenannte Bedeutung besitzen, mittels einer katalytischen Oxidationsreaktion verknüpfen. Solche katalytischen Oxidationsreaktionen gehören zum Stand der Technik und sind z.B. in der US-A-3 828 071 oder US-A-4 000 135 beschrieben.

Eine andere Möglichkeit besteht z.B. darin, daß man ein Piperazinderivat der Formel III mit einem Benzophenon der Formel V

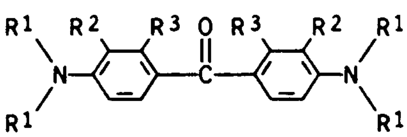 (V),

in der $R^1$, $R^2$ und $R^3$ jeweils die obengenannte Bedeutung besitzen, nach an sich bekannten Methoden kondensiert.

Eine weitere Aufgabe der vorliegenden Erfindung bestand darin, neue Piperazinderivate bereitzustellen, die sich in vorteilhafter Weise als Zwischenprodukte für die Synthese der Triphenylmethanfarbstoffe der Formel I eignen.

Demgemäß wurden die Piperazinderivate der Formel II

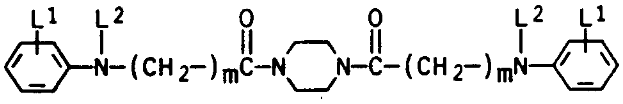 (II)

gefunden, in der die Reste

- $L^1$ gleich oder verschieden sind und unabhängig voneinander jeweils Wasserstoff, $C_1$-$C_4$-Alkyl, $C_1$-$C_4$-Alkoxy oder Halogen,
- $L^2$ gleich oder verschieden sind und unabhängig voneinander jeweils Wasserstoff oder $C_1$-$C_4$-Alkyl und
- m gleich oder verschieden ist und 1 oder 2 bedeuten,

mit der Maßgabe, daß wenn beide Reste $L^2$ gleich sind und für Wasserstoff, Methyl oder Ethyl und m jeweils für 1 stehen, beide Reste $L^1$ nicht gleichzeitig Wasserstoff bedeuten, und daß wenn beide Reste $L^2$ für Wasserstoff und m jeweils für 1 stehen, beide Reste $L^1$ nicht gleichzeitig ortho-ständiges Chlor bedeuten.

Diejenigen Verbindungen der Formel II, die definitionsgemäß ausgeschlossen sind, sind bekannt und in J. Med. Chem. Band 11, Seiten 801 bis 804, 1968, als Pharmazeutika mit analgetischem Effekt beschrieben. Über eine mögliche Eignung als Zwischenprodukte für die Synthese von Triphenylmethanfarbstoffen wird dort keine Aussage gemacht.

Die erfindungsgemäßen Piperazinderivate der Formel II können z.B. nach dem in J. Med. Chem. (loc. cit.) beschriebenen Verfahren aus Acylpiperazinen der Formel VI

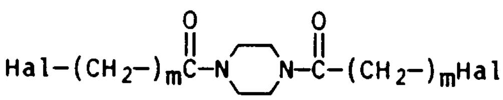 (VI),

in der Hal für Halogen steht und m die obengenannte Bedeutung besitzt, mit Anilinen der Formel VII

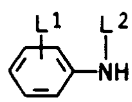 (VII),

in der $L^1$ und $L^2$ jeweils die obengenannte Bedeutung besitzen, erhalten werden.

Bei den neuen Piperazinderivaten der Formel II handelt es sich um wertvolle Zwischenprodukte für die Herstellung von Triphenylmethanfarbstoffen der Formel I.

Die neuen Triphenylmethanfarbstoffe der Formel I sind violett bis rotstichig blau. Sie können in vorteilhafter Weise als basische Farbstoffe, insbesondere zum Färben von Papier verwendet werden. Die Farbstoffe haben eine beträchtliche Substantivität und ziehen aus wäßrigem Färbemedium weitgehend aus; sie sind daher sehr umweltfreundlich.

Die folgenden Beispiele sollen die Erfindung näher erläutern.

Beispiel 1

12 g 1,4-Bis(chloracetyl)piperazin und 2,5 g Magnesiumoxid wurden in 50 ml Wasser auf 90 °C erwärmt und innerhalb einer Stunde mit 14,8 g N-Ethyl-3-methylanilin versetzt. Nach vierstündigem Erwärmen auf 100 °C wurde mit 100 ml Wasser verdünnt und mit konz. Salzsäure ein pH-Wert von 3 bis 3,5 eingestellt. Nach Abkühlung auf Raumtemperatur wurde das kristalline Produkt der Formel

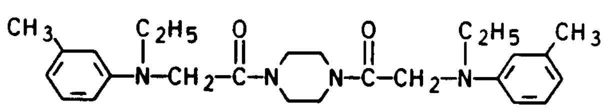

abgesaugt, mit 175 ml Wasser gewaschen und getrocknet.
Ausbeute: 20 g; Schmelzpunkt 217-220 °C

Beispiel 2

408 g 1,4-Bis(N-phenyl-N-ethylaminoacetyl)piperazin und 508 g Bis(N,N-dimethylaminophenyl)methan wurden in 1500 g Eisessig und 300 g 1,2-Propylenglykol zunächst für 10 Minuten auf 100 °C erwärmt und dann auf 44 bis 46 °C abgekühlt. (Diese Temperatur wurde während der restlichen Reaktionszeit beibehalten.) Nach Zugabe von 10 g Chloranil und 10 g der Eisenkomplexes von Dibenzotetraaza-[14]-annulen wurde unter kräftigem Rühren während 8 Stunden Luft (200 l/h) durch die Lösung geleitet. Nach Filtration erhielt man 2198 g einer ca. 38 gew.%igen Lösung des Farbstoffs der Formel

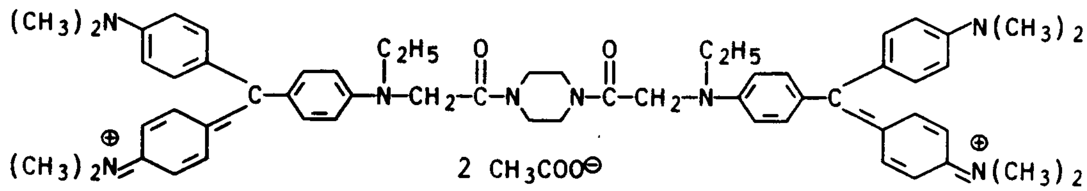

$\lambda_{max}$ (Ethanol): 589 nm, $\epsilon = 1,78 \cdot 10^5$.

Beispiel 3

44 g des in Beispiel 1 beschriebenen Produkts und 59 g Bis(4-Ethylamino-3-methylphenyl)methan wurden in 200 g Eisessig und 40 g 1,2-Propylenglykol zunächst kurzzeitig auf 95 bis 100 °C erwärmt und dann auf 44 bis 46 °C abgekühlt. (Diese Temperatur wurde während der restlichen Reaktionszeit beibehalten.) Nach Zugabe von 1 g Chloranil und 1 g des Eisenkomplexes von Dibenzotetraaza-[14]-annulen wurde die Lösung unter Sauerstoffatmosphäre (ca. 50 mb hydrostatischer Überdruck) 1 Stunde kräftig gerührt. Dabei wurden 4,6 l Sauerstoff aufgenommen. Nach Filtration erhielt man 305 g einer ca. 31 gew.%igen Lösung des Farbstoffs der Formel

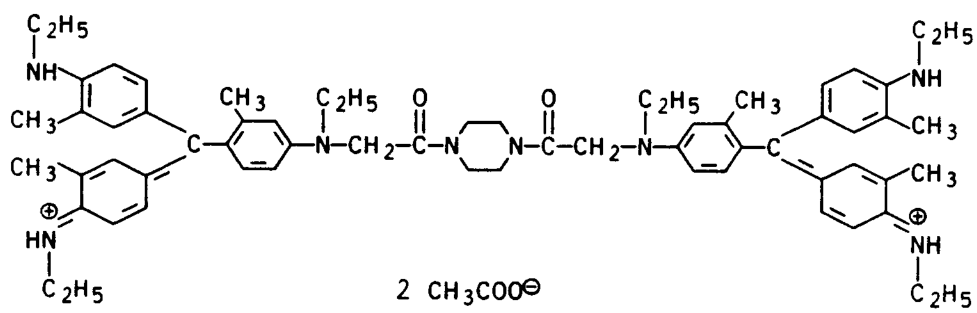

$\lambda_{max}$ (Ethanol): 587 nm, $\epsilon = 1,76 \cdot 10^5$.

In analoger Weise werden die in der folgenden Tabelle aufgeführten Farbstoffe der Formel

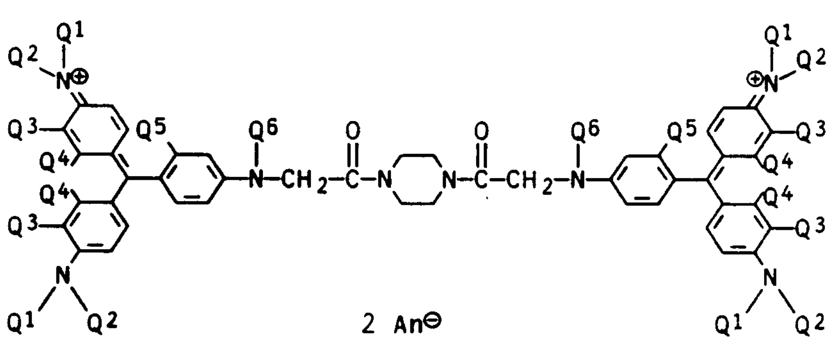

erhalten.

| Bsp. Nr. | $Q^1$ | $Q^2$ | $Q^3$ | $Q^4$ | $Q^5$ | $Q^6$ | $An^{\ominus}$ |
|---|---|---|---|---|---|---|---|
| 4 | $CH_3$ | $CH_3$ | H | H | $CH_3$ | | $CH_3COO^{\ominus}$ |
| 5 | $C_2H_5$ | H | $CH_3$ | H | H | | $CH_3COO^{\ominus}$ |
| 6 | $CH_3$ | $C_2H_5$ | H | H | H | | $CH_3COO^{\ominus}$ |
| 7 | $C_2H_5$ | $C_2H_5$ | H | OH | H | | $CH_3COO^{\ominus}$ |
| 8 | $C_2H_5$ | $C_2H_5$ | H | $OCH_3$ | H | | $CH_3COO^{\ominus}$ |
| 9 | $C_2H_5$ | $C_2H_5$ | H | $CH_3$ | H | | $CH_3COO^{\ominus}$ |
| 10 | $C_2H_4CN$ | $CH_3$ | H | H | H | | $CH_3COO^{\ominus}$ |
| 11 | $C_2H_4Cl$ | $CH_3$ | H | H | H | | $CH_3COO^{\ominus}$ |
| 12 | $C_2H_4OH$ | $CH_3$ | H | H | H | | $CH_3COO^{\ominus}$ |
| 13 | $C_2H_4OH$ | $CH_3$ | H | H | $CH_3$ | | $CH_3COO^{\ominus}$ |

## Patentansprüche

**1.** Triphenylmethanfarbstoffe der Formel I

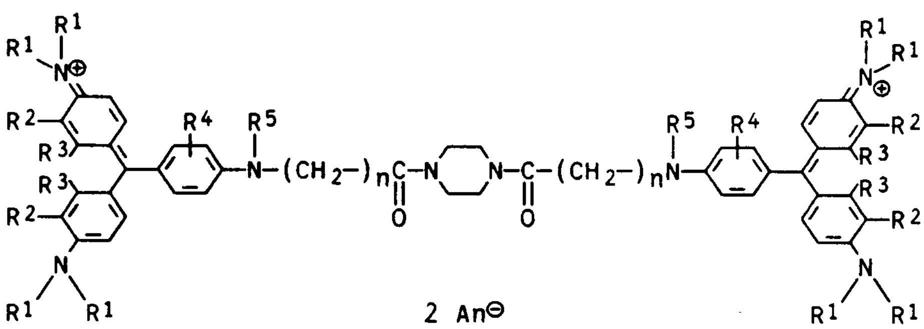

2 $An^{\ominus}$

(I),

in der die Reste

- $R^1$ gleich oder verschieden sind und unabhängig voneinander jeweils Wasserstoff oder gegebenenfalls substituiertes $C_1$-$C_6$-Alkyl oder zwei Reste $R^1$ zusammen mit dem sie verbindenen Stickstoffatom einen gesättigten 5- oder 6-gliedrigen heterocyclischen Rest, der gegebenenfalls weitere Heteroatome aufweisen kann,
- $R^2$ und $R^5$ gleich oder verschieden sind und unabhängig voneinander jeweils Wasserstoff oder $C_1$-$C_4$-Alkyl,
- $R^3$ und $R^4$ gleich oder verschieden sind und unabhängig voneinander jeweils Wasserstoff, $C_1$-$C_4$-Alkyl, $C_1$-$C_4$-Alkoxy oder Halogen,
- $An^{\ominus}$ das Äquivalent eines Anions und
- n gleich oder verschieden ist und 1 oder 2 bedeuten.

**2.** Triphenylmethanfarbstoffe nach Anspruch 1, dadurch gekennzeichnet, daß die Reste

- $R^1$ unabhängig voneinander Wasserstoff, $C_1$-$C_4$-Alkyl oder 2-Hydroxyethyl
- $R^2$ unabhängig voneinander Wasserstoff oder Methyl,
- $R^3$ und $R^4$ unabhängig voneinander Wasserstoff, Methyl, Methoxy, Ethoxy oder Halogen und

$R^5$ unabhängig voneinander Wasserstoff oder $C_1$-$C_4$-Alkyl bedeuten und $An^{\ominus}$ und n jeweils die in Anspruch 1 genannte Bedeutung besitzen.

**3.** Piperazinderivate der Formel II

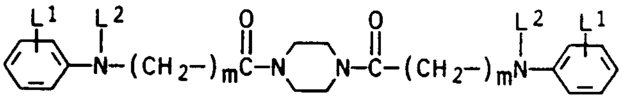

(II),

in der die Reste

- $L^1$ gleich oder verschieden sind und unabhängig voneinander jeweils Wasserstoff, $C_1$-$C_4$-Alkyl, $C_1$-$C_4$-Alkoxy oder Halogen und
- $L^2$ gleich oder verschieden sind und unabhängig voneinander jeweils Wasserstoff oder $C_1$-$C_4$-Alkyl und
- m gleich oder verschieden ist und 1 oder 2 bedeuten,

mit der Maßgabe, daß wenn beide Reste $L^2$ gleich sind und für Wasserstoff, Methyl oder Ethyl und m jeweils für 1 stehen, beide Reste $L^1$ nicht gleichzeitig Wasserstoff bedeuten, und daß wenn beide Reste $L^2$ für Wasserstoff und m jeweils für 1 stehen, beide Reste $L^1$ nicht gleichzeitig ortho-ständiges Chlor bedeuten.

**4.** Verwendung der Triphenylmethanfarbstoffe gemäß Anspruch 1 zum Färben von Papier.

**Claims**

**1.** A triphenylmethane dye of the formula I

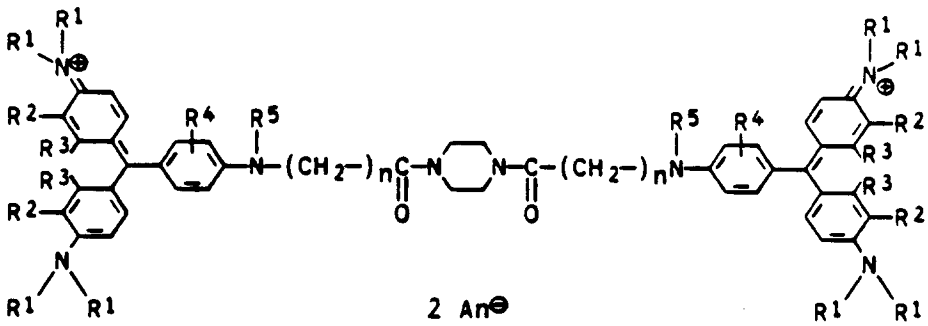

(I)

where each

- $R^1$ is identical or different, being independently of the others hydrogen or substituted or unsubstituted $C_1$-$C_6$-alkyl, or two $R^1$ radicals together with the nitrogen atom joining them form a saturated 5- or 6-membered heterocyclic radical which may contain further hetero atoms,
- $R^2$ and $R^5$ is identical or different, being independently of the others hydrogen or $C_1$-$C_4$-alkyl,
- $R^3$ and $R^4$ is identical or different, being independently of the others hydrogen, $C_1$-$C_4$-alkyl, $C_1$-$C_4$-alkoxy or halogen,
- $An^{\ominus}$ is one equivalent of an anion, and
- n is identical or different, being 1 or 2.

**2.** A triphenylmethane dye as claimed in claim 1, wherein each

- $R^1$ is independently of the others hydrogen, $C_1$-$C_4$-alkyl or 2-hydroxyethyl,
- $R^2$ is independently of the others hydrogen or methyl,
- $R^3$ and $R^4$ is independently of the others hydrogen, methyl, methoxy, ethoxy or halogen, and
- $R^5$ is independently of the other hydrogen or $C_1$-$C_4$-alkyl, and $An^{\ominus}$ and n are each as defined in claim 1.

**3.** A piperazine derivative of the formula II

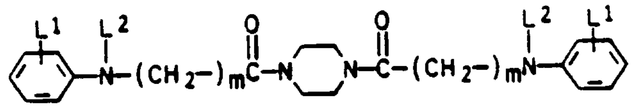 (II)

where each

- $L^1$ is identical or different, being independently of the other hydrogen, $C_1$-$C_4$-alkyl, $C_1$-$C_4$-alkoxy or halogen,
- $L^2$ is identical or different, being independently of the other hydrogen or $C_1$-$C_4$-alkyl, and
- m is identical or different, being 1 or 2, with the proviso that, if $L^2$ is the same in both cases with each $L^2$ being hydrogen, methyl or ethyl and if each m is 1, then $L^1$ is not hydrogen in both cases and that, if $L^2$ is hydrogen in both cases and each m is 1, then $L^1$ is not ortho-disposed chlorine in both cases.

**4.** A method of using a triphenylmethane dye as claimed in claim 1 for coloring paper.

**Revendications**

**1.** Colorants de la famille du triphénylméthane de formule I

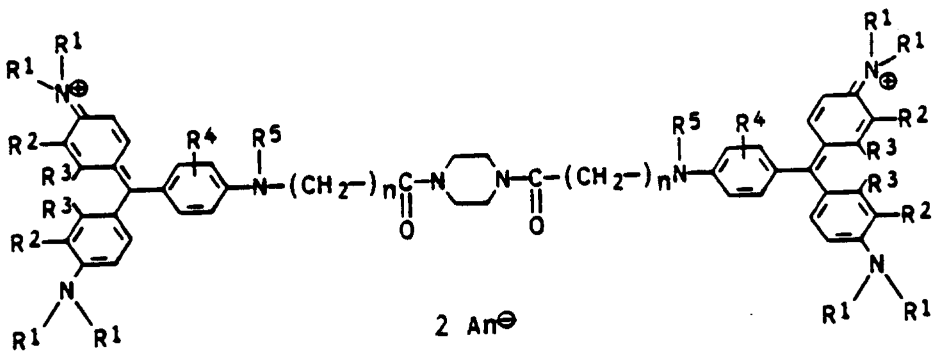 (I),

dans laquelle les restes

- $R^1$ sont identiques ou différents et représentent chacun, indépendamment les uns des autres, un atome d'hydrogène ou un radical alkyle en $C_1$-$C_6$ éventuellement substitué, ou deux restes $R^1$ représentent ensemble, avec l'atome d'azote qui les relie, un radical hétérocyclique saturé à 5 ou 6 maillons qui peut éventuellement comporter d'autres hétéroatomes,
- $R^2$ et $R^5$ sont identiques ou différents et représentent chacun, indépendamment les uns des autres, un atome d'hydrogène ou un radical alkyle en $C_1$-$C_4$,
- $R^3$ et $R^4$ sont identiques ou différents et représentent chacun, indépendamment les uns des autres, un atome d'hydrogène, d'halogène ou un radical alkyle en $C_1$-$C_4$ ou alcoxy en $C_1$-$C_4$,
- $An^{\ominus}$ est l'équivalent d'un anion et

les indices n, identiques ou différents, sont mis pour 1 ou 2.

**2.** Colorants de la famille du triphénylméthane selon la revendication 1, caractérisés en ce que les restes

- $R^1$ représentent chacun, indépendamment les uns des autres, un atome d'hydrogène ou un radical alkyle en $C_1$-$C_4$ ou 2-hydroxyéthyle,
- $R^2$ représentent chacun, indépendamment les uns des autres, un atome d'hydrogène ou un radical méthyle,
- $R^3$ et $R^4$ représentent chacun, indépendamment les uns des autres, un atome d'hydrogène, d'halogène ou un radical méthyle, méthoxy ou éthoxy,
- $R^5$ représentent chacun, indépendamment l'un de l'autre, un atome d'hydrogène au un radical alkyle en $C_1$-$C_4$ et

$An^{\ominus}$ et n ont chacun la signification donnée dans la revendication 1.

**3.** Dérivés de pipérazine de formule II

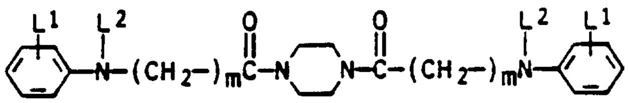

(II),

dans laquelle les restes

- $L^1$ sont identiques ou différents et représentent chacun, indépendamment l'un de l'autre, un atome d'hydrogène, d'halogène ou un radical alkyle en $C_1$-$C_4$ ou alcoxy en $C_1$-$C_4$,
- $L^2$ sont identiques ou différents et représentent chacun, indépendamment l'un de l'autre, un atome d'hydrogène ou un radical alkyle en $C_1$-$C_4$ et

les indices m sont identiques ou différents et sont mis pour 1 ou 2,
étant spécifié que quand les deux restes $L^2$ sont identiques et mis pour des atomes d'hydrogène ou pour des radicaux méthyle ou éthyle et que m est mis chaque fois pour 1, les deux restes $L^1$ ne représentent pas en même temps des atomes d'hydrogène, et que quand les deux restes $L^2$ sont mis pour des atomes d'hydrogène et que m est mis chaque fois pour 1, les deux restes $L^1$ ne représentent pas en même temps des atomes de chlore en position ortho.

**4.** Utilisation des colorants de la famille du triphénylméthane selon la revendication 1 pour la teinture de papier.